(19) Europäisches Patentamt
European Patent Office
Office européen des brevets

(11) EP 0 757 981 B1

(12) EUROPÄISCHE PATENTSCHRIFT

(45) Veröffentlichungstag und Bekanntmachung des Hinweises auf die Patenterteilung:
**16.02.2000 Patentblatt 2000/07**

(51) Int Cl.$^7$: **C07C 233/13**, C07C 235/06, C07B 55/00, C07C 211/29, C07C 209/62

(21) Anmeldenummer: **96112061.5**

(22) Anmeldetag: **26.07.1996**

(54) **Verfahren zur Herstellung von racemischen Amino-Derivaten**

Process for the preparation of racemic amino derivatives

Procédé pour la préparation de dérivés d'amino racémiques

(84) Benannte Vertragsstaaten:
**BE CH DE FR GB IT LI NL**

(30) Priorität: **09.08.1995 DE 19529293**

(43) Veröffentlichungstag der Anmeldung:
**12.02.1997 Patentblatt 1997/07**

(73) Patentinhaber: **BAYER AG**
**51368 Leverkusen (DE)**

(72) Erfinder:
- **Stelzer, Uwe, Dr.**
  **51399 Burscheid (DE)**
- **Jansen, Johannes, Rudolf, Dr.**
  **40789 Monheim (DE)**

(56) Entgegenhaltungen:
**DE-A- 4 038 356** **FR-A- 2 027 209**
**FR-A- 2 387 929**

- **TETRAHEDRON, Bd. 44, Nr. 1, 1988, Seiten 203-208, XP002014784 E. DOMINGUEZ ET AL:**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 757 981 B1

## Beschreibung

Die vorliegende Erfindung betrifft ein neues Verfahren zur Herstellung von racemischen Amino-Derivaten durch Racemisierung von optisch aktiven Amiden.

Es ist bereits bekannt geworden, optisch aktive Amide herzustellen, indem man racemische Amine enantioselektiv acyliert und das entstehende Gemisch aus optisch aktivem Amin und optisch aktivem Amid (= acyliertes Amin) trennt (vgl. DE-A 43 32 738, Chimia 48, 570 (1994)). Weiterhin ist schon bekannt, daß optisch aktive Amide durch enantioselektive Hydrolyse racemischer Amide und anschließende Trennung des anfallenden Gemisches aus optisch aktivem Amid und optisch aktivem Amin zugänglich sind (vgl. EP-A 0 399 589). Um diese Verfahren zur Racematspaltung wirtschaftlich zu gestalten, muß das jeweils unerwünschte Enantiomere wieder racemisiert und in den Kreislauf zurückgeführt werden. Ist die nicht benötigte Komponente ein optisch aktives Amin, so kann die Racemisierung durch Behandlung mit Alkanolaten in Gegenwart von Dimethylsulfoxid vorgenommen werden (vgl. DE-A 40 38 356). Handelt es sich bei dem unerwünschten Isomeren jedoch um ein Amid, so besteht ein Nachteil der Aufarbeitung darin, daß zunächst eine Verseifung des optisch aktiven Amids erforderlich ist und die Racemisierung des dabei entstehenden optisch aktiven Amins erst in einem weiteren Schritt erfolgen kann.

Es wurde nun gefunden, daß man racemische Amino-Derivate der Formel

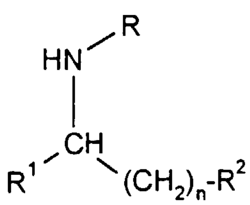

(I),

in welcher

n für die Zahlen 0, 1 oder 2 steht,

$R^1$ für gegebenenfalls substituiertes Alkyl, gegebenenfalls substituiertes Cycloalkyl, gegebenenfalls substituiertes Cycloalkenyl, gegebenenfalls substituiertes Aryl oder gegebenenfalls substituiertes Heterocyclyl steht,

$R^2$ für gegebenenfalls substituiertes Alkyl, gegebenenfalls substituiertes Cycloalkyl, gegebenenfalls substituiertes Cycloalkenyl, gegebenenfalls substituiertes Aryl oder gegebenenfalls substituiertes Heterocyclyl steht, wobei jedoch $R^1$ und $R^2-(CH_2)_n-$ nicht identisch sind, und

R für Wasserstoff oder $-CO-R^3$ steht, worin

- $R^3$ für Wasserstoff, Amino, für gegebenenfalls substituiertes Alkyl oder für gegebenenfalls substituiertes Alkoxy steht, wobei die Kohlenstoffkette bei denjenigen Resten, die mehr als ein Kohlenstoffatom enthalten, durch Heteroatome oder -gruppen, unterbrochen sein kann, oder

- $R^3$ für gegebenenfalls substituiertes Cycloalkyl, gegebenenfalls substituiertes Cycloalkenyl, gegebenenfalls substituiertes Aryl oder gegebenenfalls substituiertes Heterocyclyl steht,

erhält, wenn man optisch aktive Amide der Formel

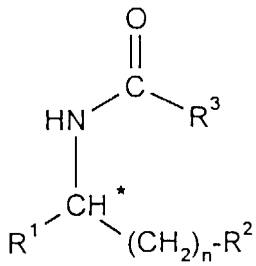

(II),

in welcher

$R^1$, $R^2$, $R^3$ und n die oben angegebenen Bedeutungen haben,

mit starken Basen aus der Gruppe der Erdalkalimetall- oder Alkalimetallhydride, -hydroxide, -amide oder alkoholate, gegebenenfalls in Gegenwart eines organischen Verdünnungsmittels umsetzt und gegebenenfalls die dabei entstehenden racemischen Amide der Formel

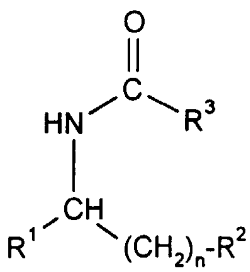

(Ia),

in welcher

$R^1$, $R^2$, $R^3$ und n die oben angegebenen Bedeutungen haben,

entweder

a) mit Wasser in Gegenwart einer Base oder einer Säure und gegebenenfalls in Gegenwart eines organischen Verdünnungsmittels umsetzt,

oder

b) sofern es sich bei den Verbindungen der Formel (Ia) um Amide handelt, in denen $R^3$ für Wasserstoff steht, mit Alkalimetallhydroxid oder Erdalkalimetallhydroxid umsetzt.

Es ist als äußerst überraschend zu bezeichnen, daß sich nach dem erfindungsgemäßen Verfahren racemische Amide und Amine herstellen lassen, denn aus dem Stand der Technik war bisher nicht bekannt, daß eine Racemisierung optisch aktiver Amide ohne vorherige Verseifung möglich ist.

Das erfindungsgemäße Verfahren zeichnet sich durch eine Reihe von Vorteilen aus. So ermöglicht es die Herstellung racemischer Amide oder Amine aus den jeweils unerwünschten Enantiomeren in hoher Ausbeute. Günstig ist auch, daß die als Ausgangsprodukte benötigten optisch aktiven Amide in einfacher Weise zugänglich sind und der Acyl-Rest breit variierbar ist. Schließlich bereitet auch die Durchführung der Umsetzung und die Isolierung der gewünschten Substanzen keinerlei Schwierigkeiten.

Alkyl steht im vorliegenden Fall, wenn nicht anders definiert, für gesättigte aliphatische Kohlenwasserstoff-Reste, die geradkettig oder verzweigt sein können.

Cycloalkyl steht im vorliegenden Fall, wenn nicht anders definiert, für gesättigte, carbocyclische Reste, die gegebenenfalls mit weiteren ankondensierten oder überbrückten Ringen ein bi- oder polycyclisches Ringsystem bilden.

Cycloalkenyl steht im vorliegenden Fall, wenn nicht anders definiert, für ungesättigte, carbocyclische Reste, die gegebenenfalls mit weiteren ankondensierten oder überbrückten Ringen ein bi- oder polycyclisches Ringsystem bilden.

Aryl steht im vorliegenden Fall, wenn nicht anders definiert, für aromatische, mono-, bi- oder polycyclische

Kohlenwasserstoffreste, wie z. B. Phenyl, Naphthyl, Anthranyl, Phenanthryl, vorzugsweise Phenyl oder Naphthyl, insbesondere Phenyl.

Heterocyclyl steht im vorliegenden Fall, wenn nicht anders definiert, für gesättigte oder ungesättigte, ringförmige Reste, in denen mindestens ein Ringglied ein Heteroatom, d. h. ein von Kohlenstoff verschiedenes Atom, ist. Enthält der Ring mehrere Heteroatome, so können diese gleich oder verschieden sein. Heteroatome sind bevorzugt Sauerstoff, Stickstoff oder Schwefel. Gegebenenfalls bilden die ringförmigen Reste mit weiteren carbocyclischen oder heterocyclischen, ankondensierten oder überbrückten Ringen gemeinsam ein bi- oder polycyclisches Ringsystem. Bevorzugt sind mono- oder bicyclische Ringsysteme, insbesondere mono- oder bicyclische Ringsysteme mit aromatischem Charakter.

In den Definitionen sind die gesättigten oder ungesättigten Kohlenstoffketten, wie Alkyl, Alkandiyl, Alkenyl oder Alkinyl, auch in Verknüpfung mit Gruppen, die über Heteroatome verbunden sind, also Gruppen, wie Alkoxy, Alkylthio oder Alkylamino, jeweils geradkettig oder verzweigt.

Halogen steht im vorliegenden Fall, wenn nicht anders definiert für Fluor, Chlor, Brom oder Iod, vorzugsweise für Fluor, Chlor oder Brom, insbesondere für Fluor oder Chlor.

Verwendet man (S)-N-[1-(4-Chlorphenyl)-ethyl]-acetamid als Ausgangsstoff und Kaliumhydroxid als starke Base, so kann der Verlauf der ersten Stufe des erfindungsgemäßen Verfahrens durch das folgende Formelschema veranschaulicht werden:

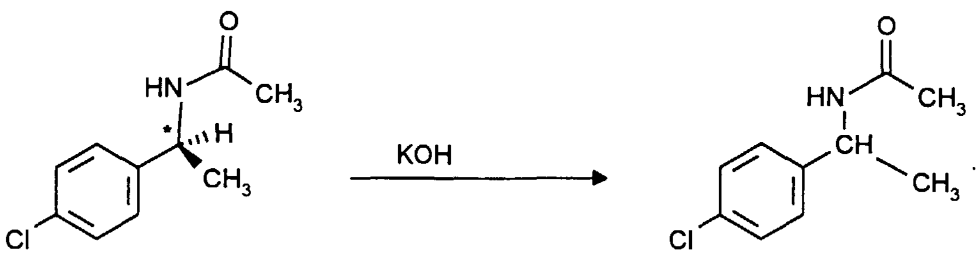


(S-Amid) (R,S-Amid)

Verwendet man racemisches N-[1-(4-Chlorphenyl)-ethyl]-acetamid als Ausgangskomponente und wäßrige Salzsäure als Reaktionskomponente, so kann der Verlauf der zweiten Stufe des erfindungsgemäßen Verfahrens durch das folgende Formelschema veranschaulicht werden:

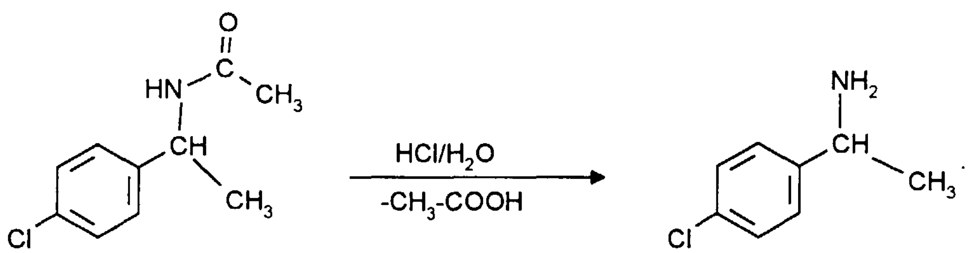


(R,S-Amid) (R,S-Amin)

Die zur Durchführung des erfindungsgemäßen Verfahrens als Ausgangsstoffe benötigten optisch aktiven Amide sind durch die Formel (II) allgemein definiert. In dieser Formel ist das asymmetrisch substituierte Kohlenstoffatom durch (*) gekennzeichnet. Auch in anderen Formeln optisch aktiver Verbindungen sind Chiralitätszentren jeweils in gleicher Weise markiert.

n steht auch vorzugsweise für die Zahlen 0, 1 oder 2.

$R^1$ steht vorzugsweise für geradkettiges oder verzweigtes Alkyl mit 1 bis 8 Kohlenstoffatomen, wobei die Alkylreste einfach oder mehrfach, gleichartig oder verschieden substituiert sein können durch Halogen, Amino, Hydroxy, Formyl, Carboxy, Carbamoyl, jeweils geradkettiges oder verzweigtes Alkoxy oder Alkylthio mit jeweils 1 bis 6 Kohlenstoffatomen;

und/oder durch jeweils geradkettiges oder verzweigtes Halogenalkoxy oder Halogenalkylthio mit jeweils 1 bis 6 Kohlenstoffatomen und 1 bis 13 gleichen oder verschiedenen Halogenatomen; und/oder durch jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen; oder durch zweifach verknüpftes Alkylen mit 1 bis 6 Kohlenstoffatomen oder durch zweifach verknüpftes Dioxyalkylen mit 1 bis 4 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder

$R^1$ steht vorzugsweise für Cycloalkyl mit 3 bis 7 Kohlenstoffatomen, wobei diese Reste einfach bis vierfach, gleichartig oder verschieden substituiert sein können durch Halogen und/oder Alkyl mit 1 bis 4 Kohlenstoffatomen, oder für Cycloalkenyl mit 3 bis 7 Kohlenstoffatomen, wobei diese Reste einfach bis vierfach, gleichartig oder verschieden substituiert sein können durch Halogen und/oder Alkyl mit 1 bis 4 Kohlenstoffatomen, oder für gesättigtes oder ungesättigtes Heterocyclyl mit 3 bis 7 Ringgliedern, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder
für Aryl mit 6 bis 10 Kohlenstoffatomen, wobei jeder dieser Reste einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen,
geradkettiges oder verzweigtes Alkoxy oder Alkylthio mit 1 bis 4 Kohlenstoffatomen, geradkettiges oder verzweigtes Halogenalkoxy oder Halogenalkylthio mit 1 bis 4 Kohlenstoffatomen und 1 bis 9 gleichen oder verschiedenen Halogenatomen, Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen.

$R^2$ steht vorzugsweise für geradkettiges oder verzweigtes Alkyl mit 1 bis 8 Kohlenstoffatomen oder

$R^2$ steht vorzugsweise für Cycloalkyl mit 3 bis 7 Kohlenstoffatomen, wobei diese Reste einfach bis vierfach, gleichartig oder verschieden substituiert sein können durch Halogen und/oder Alkyl mit 1 bis 4 Kohlenstoffatomen, oder für Cycloalkenyl mit 3 bis 7 Kohlenstoffatomen, wobei diese Reste einfach bis vierfach, gleichartig oder verschieden substituiert sein können durch Halogen und/oder Alkyl mit 1 bis 4 Kohlenstoffatomen, oder für gesättigtes oder ungesättigtes Heterocyclyl mit 3 bis 7 Ringgliedern, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder
für Aryl mit 6 bis 10 Kohlenstoffatomen, wobei jeder dieser Reste einfach bis fünffach, gleichartig oder verschieden substituiert sein kann durch Halogen, Nitro, Amino, Hydroxy, Formyl, Carboxy, Carbamoyl, Thiocarbamoyl; jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen.

$R^3$ steht vorzugsweise für Wasserstoff, Amino, oder

$R^3$ steht vorzugsweise für geradkettiges oder verzweigtes Alkyl mit 1 bis 10 Kohlenstoffatomen oder geradkettiges oder verzweigtes Alkoxy mit 1 bis 6 Kohlenstoffatomen, wobei die Alkylreste oder Alkoxyreste einfach oder mehrfach, gleichartig oder verschieden substituiert sein können durch Halogen, Nitro, Amino, Hydroxy, Formyl, Carboxy, Carbamoyl;
und/oder durch jeweils geradkettiges oder verzweigtes Alkoxy, Alkylthio, Alkylsulfinyl oder Alkylsulfonyl mit jeweils 1 bis 6 Kohlenstoffatomen; und/oder durch jeweils geradkettiges oder verzweigtes Alkenyl oder Alkenyloxy mit jeweils 2 bis 6 Kohlenstoffatomen;
und/oder durch jeweils geradkettiges oder verzweigtes Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
und/oder durch jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
oder durch zweifach verknüpftes Alkylen mit 1 bis 6 Kohlenstoffatomen oder durch zweifach verknüpftes Dioxyalkylen mit 1 bis 4 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder
durch Phenyl, oder

$R^3$ steht vorzugsweise für Cycloalkyl mit 3 bis 7 Kohlenstoffatomen, wobei diese Reste einfach bis vierfach, gleichartig oder verschieden substituiert sein können durch Halogen und/oder Alkyl mit 1 bis 4 Kohlenstoffatomen, oder
für Cycloalkenyl mit 3 bis 7 Kohlenstoffatomen, wobei diese Reste einfach bis vierfach, gleichartig oder verschieden substituiert sein können durch Halogen und/oder Alkyl mit 1 bis 4 Kohlenstoffatomen, oder
für gesättigtes oder ungesättigtes Heterocyclyl mit 3 bis 7 Ringgliedern, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder
für Aryl mit 6 bis 10 Kohlenstoffatomen, wobei jeder dieser Reste einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen,
geradkettiges oder verzweigtes Alkoxy oder Alkylthio mit 1 bis 4 Kohlenstoffatomen,
geradkettiges oder verzweigtes Halogenalkoxy oder Halogenalkylthio mit 1 bis 4 Kohlenstoffatomen und 1 bis 9 gleichen oder verschiedenen Halogenatomen,
Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen.

n steht auch besonders bevorzugt für die Zahlen 0,1 oder 2.

$R^1$ steht besonders bevorzugt für Methyl, Ethyl, n-Propyl oder Isopropyl.

$R^2$ steht besonders bevorzugt für Cyclohexyl, Norbornyl oder Cyclohexenyl, wobei diese Reste einfach bis vierfach, gleichartig oder verschieden substituiert sein können durch Fluor, Chlor, Methyl und/oder Ethyl, oder
für Furyl, Pyridyl oder Thienyl, wobei diese Reste einfach bis dreifach, gleichartig oder verschieden substituiert sein können durch Fluor, Chlor, Brom, Methyl, Ethyl, n-Propyl, i-Propyl, n-Butyl, i-Butyl, s-Butyl, t-Butyl, Methoxy,

Ethoxy, n-Propoxy, i-Propoxy, Trifluormethyl und/oder Trifluorethyl, oder für Phenyl oder Naphthyl, wobei jeder dieser Reste einfach bis fünffach, gleichartig oder verschieden substituiert sein kann durch Fluor, Chlor, Brom, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Methoxycarbonyl, Ethoxycarbonyl, Phenyl, Phenoxy, Phenylthio, Benzyloxy, Benzylthio oder Phenylethyloxy, oder durch jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy.

$R^3$ steht besonders bevorzugt für Wasserstoff, Amino, Methyl, Ethyl, n- oder i-Propyl, n-, i-, s-Butyl, Pentyl, Hexyl, Heptyl, Octyl, Methoxy, Ethoxy, Hydroxymethyl, 1-Hydroxy-1-ethyl, Methoxymcthyl, Methoxyethyl, Methoxypropyl, Ethoxymethyl, Ethoxyethyl, 2-Carboxy-1-hydroxy-1-ethyl, 2-Carboxy-1-ethyl, 3-Carboxy-1-propyl und Benzyl.

Die optisch aktiven Amide der Formel (II) sind bekannt und/oder lassen sich nach bekannten Methoden herstellen (vergleiche DE-A 4332738 und Chimia 48, 570 (1994)).

Als Verdünnungsmittel kommen bei der Durchführung der ersten Stufe des erfindungsgemäßen Verfahrens alle polaren, aprotischen, organischen Solventien in Betracht. Vorzugsweise verwendbar sind Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid, Sulfoxide, wie Dimethylsulfoxid; Sulfone, wie Sulfolan.

Als starke Basen kommen bei der Durchführung der ersten Stufe des erfindungsgemäßen Verfahrens anorganische und organische Säurebindemittel aus der Gruppe der Erdalkalimetall- oder Alkalimetallhydride, -hydroxide, -amide oder -alkoholate, wie beispielsweise Natriumhydrid, Natriumamid, Natrium-methylat, Natrium-ethylat, Kalium-tert.-butylat, Natriumhydroxid oder Kaliumhydroxid, in Frage.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 0°C und 280°C, vorzugsweise zwischen 20°C und 250°C.

Sowohl bei der Durchführung der ersten als auch der zweiten Stufe des erfindungsgemäßen Verfahrens arbeitet man im allgemeinen unter Atmosphärendruck. Es ist jedoch auch möglich, jeweils unter erhöhtem oder vermindertem Druck zu arbeiten. So kann man die Umsetzungen unter Drucken zwischen 0,1 bar und 10 bar durchführen.

Außerdem kann man sowohl bei der Durchführung der ersten als auch der zweiten Stufe des erfindungsgemäßen Verfahrens unter Inertgas-Atmosphäre arbeiten, vorzugsweise unter Stickstoff oder Argon.

Bei der Durchführung der ersten Stufe des erfindungsgemäßen Verfahrens setzt man auf 1 Mol an optisch aktivem Amid der Formel (II) im allgemeinen 0,01 bis 2 Mol, vorzugsweise 0,02 bis 1 Mol, an starker Base ein. Die Aufarbeitung erfolgt nach üblichen Methoden. Arbeitet man in Abwesenheit von Verdünnungsmitteln, so verfährt man im allgemeinen in der Weise, daß man das Reaktionsgemisch mit einem mit Wasser wenig mischbaren organischen Lösungsmittel versetzt, das entstehende Gemisch mit Wasser wäscht, dann trocknet und einengt. ArbeiteLman in Gegenwart von Verdünnungsmitteln, so geht man im allgemeinen so vor, daß man das Reaktionsgemisch einengt, den verbleibenden Rückstand mit Wasser versetzt, mit einem mit Wasser wenig mischbaren organischen Solvens extrahiert, die vereinigten organischen Phasen trocknet und einengt.

Bei der Durchführung der zweiten Stufe des erfindungsgemäßen Verfahrens nach Variante (a) kommen als Basen alle üblichen starken anorganischen und organischen Basen in Betracht. Vorzugsweise in Frage kommen diejenigen Basen, die im Zusammenhang mit der ersten Stufe des erfindungsgemäßen Verfahrens als bevorzugt genannt wurden. Außerdem kommen auch Säuren, wie verdünnte Schwefelsäure und Salzsäure in Frage.

Als Verdünnungsmittel kommen bei der Durchführung der zweiten Stufe des erfindungsgemäßen Verfahrens nach Variante (a) alle zur Verseifung von Amiden üblichen organischen Solventien in Frage. Vorzugsweise verwendbar sind Alkohole, wie Methanol, Ethanol, n-Butanol und Propanol, ferner Dimethylsulfoxid und außerdem Wasser, sowie Mischungen dieser Solventien.

Die Reaktionstemperaturen können auch bei der Durchführung der zweiten Stufe des erfindungsgemäßen Verfahrens nach Variante (a) in einem größeren Bereich variiert werden. Im allgemeinen arbeitet man bei Temperaturen zwischen 0°C und 200°C, vorzugsweise zwischen 20°C und 150°C.

Bei der Durchführung der zweiten Stufe des erfindungsgemäßen Verfahrens nach Variante (a) setzt man auf 1 Mol an racemischem Amid der Formel (Ia) im allgemeinen 0,1 bis 5 Mol, vorzugsweise 1 bis 3 Mol an Base oder Säure ein. Die Aufarbeitung erfolgt nach üblichen Methoden. Im allgemeinen geht man so vor, daß man das Reaktionsgemisch, gegebenenfalls nach vorherigem Einengen, mit einem mit Wasser wenig mischbaren organischen Solvens extrahiert, die vereinigten organischen Phasen wäscht, trocknet und einengt.

Bei der Durchführung der zweiten Stufe des erfindungsgemäßen Verfahrens nach Variante (b) kommen als Hydroxide vorzugsweise Natriumhydroxid, Kaliumhydroxid oder Calciumhydroxid in Betracht.

Die Reaktionstemperaturen können auch bei der Durchführung der zweiten Stufe des erfindungsgemäßen Verfahrens nach Variante (b) innerhalb eines größeren Bereiches variiert werden. Im allgemeinen arbeitet man bei Temperaturen zwischen 40°C und 220°C, vorzugsweise zwischen 40°C und 180°C.

Bei der Durchführung der zweiten Stufe des erfindungsgemäßen Verfahrens nach Variante (b) setzt man auf 1 Mol an racemischem Amid der Formel (Ia) im allgemeinen 0,02 bis 5 Mol an Alkalimetallhydroxid bzw. eine entsprechende Menge an Erdalkalimetallhydroxid ein. Die Aufarbeitung erfolgt nach üblichen Methoden. Im allgemeinen verfährt man in der Weise, daß man das Reaktionsgemisch mit Wasser versetzt, dann mit einem mit Wasser wenig mischbaren organischen Lösungsmittel extrahiert und die vereinigten organischen Phasen trocknet und einengt.

In einer bevorzugten Ausführungsform verfährt man bei dem erfindungsgemäßen Verfahren in der Weise, daß man auf 1 Mol an optisch aktivem Amid der Formel (II) im allgemeinen 0,02 bis 1,0 Mol, vorzugsweise 0,05 bis 0,5 Mol, an Alkalimetallhydroxid einsetzt und das Gemisch auf Temperaturen zwischen 20°C und 280°C, vorzugsweise zwischen 50°C und 250°C, erhitzt, so daß eine Schmelze entsteht. Nach beendeter Racemisierung (nach 0,5 bis 36 Stunden) kann das entstandene racemische Amid der Formel (Ia) durch Zugabe von 20 bis 2000 ml Wasser pro Mol Amid und gegebenenfalls durch Zugabe von weiterem Alkalimetallhydroxid oder von Erdalkalimetallhydroxid zum racemischen Amin verseift werden, indem man auf Temperaturen zwischen 20°C und 100°C erhitzt.

Eine weitere bevorzugte Ausführungsform des erfindungsgemäßen Verfahrens besteht darin, daß man auf 1 Mol an optisch aktivem Amid der Formel (II) 0,02 bis 1,0 Mol, vorzugsweise 0,05 bis 0,5 Mol, eines Alkalimetallhydrides, vorzugsweise Natriumhydrid, einsetzt und das Gemisch auf Temperaturen zwischen 20°C und 280°C, vorzugsweise zwischen 50°C und 250°C, erhitzt, so daß eine Schmelze entsteht. Gegebenenfalls kann eine Verseifung des Amids angeschlossen werden.

In einer weiteren bevorzugten Ausführungsform verfährt man bei dem erfindungsgemäßen Verfahren in der Weise, daß man auf 1 Mol an optisch aktivem Amid der Formel (II) 0,02 bis 1,0 Mol, vorzugsweise 0,05 bis 0,5 Mol, eines Alkalimetall- oder Erdalkalimetallalkoholates, vorzugsweise Kalium-tert.-butanolat einsetzt und in Gegenwart eines polaren, aprotischen, organischen Lösungsmittels arbeitet. Als Solventien verwendbar sind dabei vorzugsweise Nitrile, wie Acetonitril, Propionitril, n- oder i-Butyronitril oder Benzonitril; ferner Amide, wie N,N-Dimethylformamid, N, N-Dimethylacetamid, N-Methylformanilid, N-Methylpyrrolidon oder Hexamethylphosphorsäuretriamid; außerdem Sulfoxide, wie Dimethylsulfoxid; und auch Sulfone, wie Sulfolan, gegebenenfalls auch in Mischung mit Alkoholen, wie tert.-Butanol.

Eine Verseifung des Amids kann danach erfolgen.

In einer anderen bevorzugten Ausführungsform geht man bei dem erfindungsgemäßen Verfahren so vor, daß man auf 1 Mol an optisch aktivem Amid der Formel (II) im allgemeinen 0,02 bis 1,0 Mol, vorzugsweise 0,05 bis 0,5 Mol, eines Metallalkoholates, vorzugsweise eines Alkali-, Erdalkali- oder Erdmetallalkoholates, insbesondere Kalium-tert.-butanolat, einsetzt und das Gemisch auf Temperaturen zwischen 40°C und 220°C erhitzt, so daß eine Schmelze entsteht. Handelt es sich bei dem Amid der Formel (II) um eine Verbindung, in der $R^3$ für Wasserstoff steht, so kann nach dem Racemisierungsschritt durch Zugabe von 0,02 bis 5 Mol eines Alkalimetall- oder Erdalkalimetall-hydroxids, wie Natriumhydroxid, Kaliumhydroxid oder Calciumhydroxid, pro Mol an Amid und erneutes Erhitzen auf Temperaturen zwischen 40°C und 220°C (0,5 bis 24 Stunden) direkt das entsprechende racemische Amin erhalten werden.

In einer weiteren bevorzugten Ausführungsform des erfindungsgemäßen Verfahrens geht man so vor, daß man auf 1 Mol an optisch aktivem Amid der Formel (II) im allgemeinen 0,02 bis 1 Mol, vorzugsweise 0,05 bis 0,5 Mol, eines Alkali-, Erdalkali- oder Erdmetallhydroxids, insbesondere Kaliumhydroxid, einsetzt und in Gegenwart eines polaren, aprotischen, organischen Lösungsmittels arbeitet. Als Solventien verwendbar sind dabei vorzugsweise Nitrile, wie Acetonitril, Propionitril, n- oder i-Butyronitril oder Benzonitril; außerdem Amide, wie N,N-Dimethylformamid, N,N-Dimethylacetamid, N-Methylformanilid, N-Methylpyrrolidon oder Hexamethylphosphorsäuretriamid; ferner Sulfoxide, wie Dimethylsulfoxid; sowie Sulfone, wie Sulfolan, gegebenenfalls auch in Mischung mit Alkoholen, wie tert.-Butanol.

Eine Verseifung des Amids kann angeschlossen werden.

Die nach dem erfindungsgemäßen Verfahren erhältlichen racemischen Amide und Amine können entweder direkt oder nach vorheriger Racematspaltung als Zwischenprodukte für weitere Synthesen eingesetzt werden. Vor allem (R)-Amine der Formel (I) sind wertvolle Zwischenprodukte zur Herstellung-von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475). So erhält man zum Beispiel die fungizid wirksame Verbindung der Formel

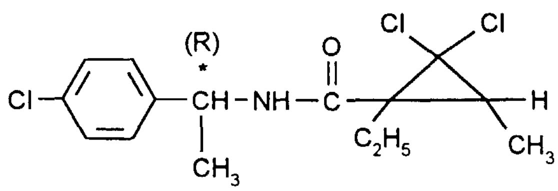

(III),

indem man das (R)-1-(4-Chlor-phenyl)-ethylamin der Formel

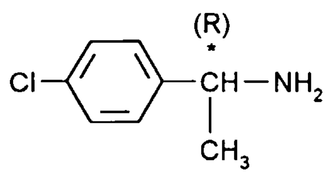

(IV)

mit 2,2-Dichlor-1-ethyl-3-methyl-1-cyclopropancarbonsäurechlorid der Formel

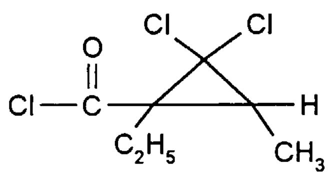

(V)

in Gegenwart eines Säurebindemittels und in Gegenwart eines inerten organischen Verdünnungsmittels umsetzt.

Die Durchführung des erfindungsgemäßen Verfahrens wird durch die folgenden Beispiele veranschaulicht.

## Herstellungsbeispiele

### Beispiel 1

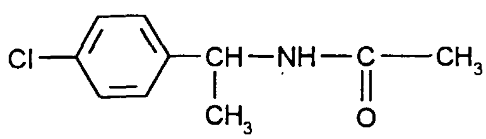

(I-1)

Jeweils 4 g (0,020 Mol) (S)-N-[1-(4-Chlorphenyl)-ethyl]-acetamid werden mit den in den Tabellen 1 bis 3 angegebenen Mengen an Kaliumhydroxid für die jeweils angegebene Zeit auf 130 bis 140°C erhitzt, so daß eine Schmelze entsteht. Der Verlauf der Umsetzung wird dabei durch Entnahme von Proben kontrolliert. Als Maß für den Racemisierungsgrad dient der ee-Wert, der den Enantiomeren-Überschuß angibt und sich wie folgt berechnet:

$$ee = \frac{(R - S)}{(R + S)} \times 100\ \%.$$

Dabei bezeichnen R und S die Konzentration der einzelnen Enantiomeren des gebildeten Amids.

Nach beendeter Umsetzung läßt man die Schmelze abkühlen, gibt jeweils 100 ml Methyl-tert.-butyl-ether zu, wäscht das entstehende Gemisch mit Wasser, trocknet die organische Phase über Natriumsulfat und engt unter vermindertem Druck ein. Man erhält auf diese Weise racemisches N-[1-(4-Chlor-phenyl)-ethyl]-acetamid in Ausbeuten zwischen 95 und 100 %.

Tabelle 1

| Eingesetzte Menge an Kaliumhydroxid: 20 Mol-% | | | | | | | | |
|---|---|---|---|---|---|---|---|---|
| ee-Wert (%) (S)-N-[1-[4-Chlorphenyl)-ethyl]-acetamid | 63,3 | 39,9 | 23,9 | 15,4 | 9,04 | 5,65 | 3,86 | 1,28 |
| Reaktionszeit (Stunden) | 0 | 0,5 | 1 | 1,5 | 2 | 2,5 | 3 | 4 |

Tabelle 2

| Eingesetzte Menge an Kaliumhydroxid: 10 Mol-% | | | | | | | | |
|---|---|---|---|---|---|---|---|---|
| ee-Wert (%) (S)-N-[1-[4-Chlorphenyl)-ethyl]-acetamid | 63,5 | 49,8 | 37,8 | 30,5 | 20,2 | 14,8 | 7,5 | 4 |
| Reaktionszeit (Stunden) | 0 | 0,5 | 1 | 2 | 3 | 4 | 6 | 12 |

Tabelle 3

| Eingesetzte Menge an Kaliumhydroxid: 50 Mol-% | | | | | | | | |
|---|---|---|---|---|---|---|---|---|
| ee-Wert (%) (S)-N-[1-[4-Chlorphenyl)-ethyl]-acetamid | 63 | 36,3 | 22,1 | 14,2 | 10 | 4,8 | 2,9 | 2 |
| Reaktionszeit (Stunden) | 0 | 0,5 | 1 | 1,5 | 2 | 3 | 4 | 6 |

**Beispiel 2**

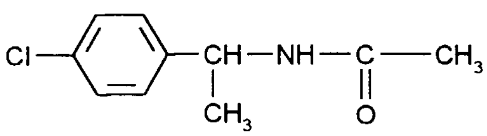

(I-1)

Ein Gemisch aus 9,87 g (0,05 Mol) (S)-N-[1-(4-Chlor-phenyl)-ethyl]acetamid mit einem ee-Wert von 64 % und 0,15 g (0,005 Mol) Natriumhydroxid wird unter Argon 20 Stunden auf 140 bis 150°C erhitzt. Danach läßt man die Schmelze abkühlen, gibt 200 ml Wasser zu, extrahiert mit Methyl-tert.-butyl-ether, trocknet die vereinigten organischen Phasen über Natriumsulfat und engt unter vermindertem Druck ein. Man erhält auf diese Weise 9,45 g an N-[1-(4-Chlor-phenyl)-ethyl]-acetamid mit einem ee-Wert von 2,5 %. Die Ausbeute an racemischem Produkt errechnet sich danach zu 95,6 % der Theorie.

**Beispiel 3**

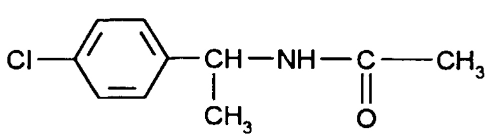

(I-1)

Ein Gemisch aus 3,95 g (0,02 Mol) (S)-N-[1-(4-Chlor-phenyl)-ethyl]-acetamid mit einem ee-Wert von 64 %, 0,45 g (0,004 Mol) Kalium-tert.-butanolat und 24 ml Dimethylsulfoxid wird 24 Stunden auf 90°C erhitzt. Danach wird das Reaktionsgemisch unter vermindertem Druck eingeengt und der verbleibende Rückstand mit 50 ml Wasser versetzt. Man extrahiert das entstehende Gemisch mit Methyl-tert.-butyl-ether, trocknet die vereinigten organischen Phasen über Natriumsulfat und engt unter vermindertem Druck ein. Man erhält auf diese Weise 3,45 g an N-[1-(4-Chlor-phenyl)-ethyl]-acetamid mit einem ee-Wert von 12,5 %. Die Ausbeute an racemischem Produkt errechnet sich danach zu 94 % der Theorie.

**Beispiel 4**

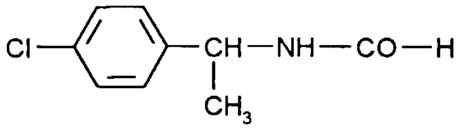

(I-2)

Ein Gemisch aus 2,76 g (0,015 Mol) (S)-N-[1-(4-Chlor-phenyl)-ethyl]-formamid, mit einem ee-Wert von 64 %, 0,37 g (3,3 mmol) Kalium-tert.-butanolat und 15 ml Dimethylsulfoxid wird 30 Stunden lang auf 90°C erhitzt. Danach wird das Reaktionsgemisch unter vermindertem Druck eingeengt und der verbleibende Rückstand mit 50 ml Wasser versetzt. Man extrahiert das entstehende Gemisch mit Methyl-tert.-butyl-ether, trocknet die vereinigten organischen Phasen über Natriumsulfat und engt unter vermindertem Druck ein. Man erhält auf diese Weise 2 g an N-[1-(4-Chlor-phenyl)-ethyl]-formamid mit einem ee-Wert von 3,9 %. Die Ausbeute an racemischem Produkt errechnet sich danach zu 74 % der Theorie.

**Beispiel 5**

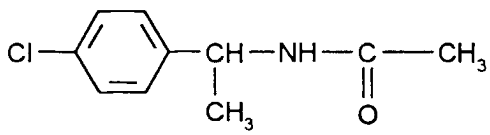

(I-1)

Ein Gemisch aus 3,95 g (0,02 Mol) (S)-N-[1-(4-Chlor-phenyl)-ethyl]-acetarnid mit einem ee-Wert von 64 % und 0,45 g (0,004 Mol) Kalium-tert.-butanolat wird 24 Stunden lang auf 150°C erhitzt. Danach läßt man die Schmelze abkühlen, gibt 50 ml Methyl-tert.-butyl-ether zu, wäscht mit Wasser, trocknet die organische Phase über Natriumsulfat und engt unter vermindertem Druck ein. Man erhält auf diese Weise 3,67 g an N-[1-(4-Chlor-phenyl)-ethyl]-acetamid mit einem ee-Wert von 0 %. Die Ausbeute an racemischem Produkt errechnet sich danach zu 90,3 % der Theorie.

**Beispiel 6**

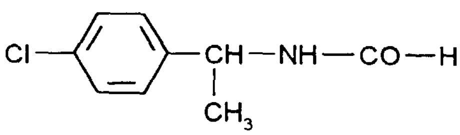

(I-2)

Ein Gemisch aus 2,76 g (0,015 Mol) (S)-N-[1-(4-Chlor-phenyl)-ethyl]-formamid mit einem ee-Wert von 64 % und 0,336 g (0,0029 Mol) Kalium-tert.-butanolat wird 20 Stunden lang auf 150°C erhitzt. Danach läßt man die Schmelze abkühlen, gibt 50 ml Methyl-tert.-butyl-ether zu, wäscht mit Wasser, trocknet die organische Phase über Natriumsulfat und engt unter vermindertem Druck ein. Man erhält auf diese Weise 2,56 g an N-[1-(4-Chlor-phenyl)-ethyl]-formamid mit einem ee-Wert von 2,6 %. Die Ausbeute an racemischem Produkt errechnet sich danach zu 93,3 % der Theorie.

**Beispiel 7**

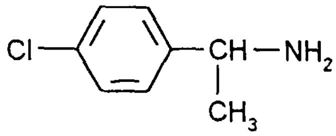

(I-3)

Ein Gemisch aus 9,5 g (0,0517 Mol) (S)-N-[1-(4-Chlor-phenyl)-ethyl]-formamid mit einem ee-Wert von 64 % und 1,55 g (0,0138 Mol) Kalium-tert.-butanolat wird 24 Stunden auf 150°C erhitzt. Danach wird abgekühlt, mit 3,75 g (0,056 Mol) gepulvertem Kaliumhydroxid versetzt und 20 Stunden auf 150°C erhitzt. Nach dem Abkühlen der Schmelze gibt man 100 ml Wasser zu, extrahiert mit Dichlormethan, trocknet die vereinigten organischen Phasen über Natriumsulfat und engt unter vermindertem Druck ein. Man erhält auf diese Weise 7,3 g an 1-(4-Chlor-phenyl)-ethyl-amin mit einem ee-Wert von 0 %. Die Ausbeute an racemischem Produkt errechnet sich danach zu 91,6 % der Theorie.

**Beispiel 8**

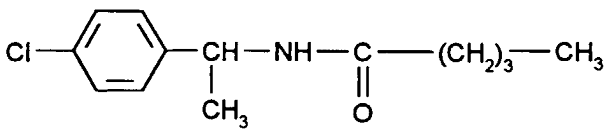

(I-4)

Ein Gemisch aus 2,4 g (0,01 Mol) (S)-N-[1-(4-Chlor-phenyl)-ethyl]-valeriansäureamid ($[\alpha]_D^{20}$ = -105,1° c = 1 in Methanol) und 1,12 g (0,01 Mol) Kalium-tert.-butanolat wird 30 Stunden auf 120°C erhitzt. Nach dem Abkühlen der Schmelze gibt man 50 ml Dichlormethan zu, wäscht mit Wasser, trocknet die organische Phase über Natriumsulfat und engt bei vermindertem Druck ein.

Man erhält auf diese Weise 1,93 g an N-[1-(4-Chlor-phenyl)-ethyl]-valeriansäureamid ($[\alpha]_D^{20}$ = ±0° c = 1,01 in Methanol). Die Ausbeute an racemischem Produkt errechnet sich danach zu 80,29 % der Theorie.

**Beispiel 9**

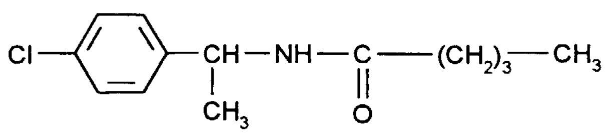

(I-4)

Ein Gemisch aus 2,4 g (0,01 Mol) (S)-N-[1-(4-Chlor-phenyl)-ethyl]-valeriansäureamid ($[\alpha]_D^{20}$ = -105,1° c = 1 in Methanol), 0,56 g (0,005 Mol) Kalium-tert.-butanolat und 20 ml Dimethylsulfoxid wird 30 Stunden lang auf 100°C erhitzt. Danach wird das Reaktionsgemisch unter vermindertem Druck eingeengt und der verbleibende Rückstand mit 50 ml Dichlormethan versetzt. Man wäscht das entstehende Gemisch dreimal mit je 20 ml Wasser, trocknet über Natriumsulfat und engt unter vermindertem Druck ein.

Man erhält auf diese Weise 2,05 g an N-[1-(4-Chlor-phenyl)-ethyl]-valeriansäureamid ($[\alpha]_D^{20}$ = ±0° c = 1,1 in Methanol). Die Ausbeute an racemischem Produkt errechnet sich danach zu 85,8 % der Theorie.

**Beispiel 10**

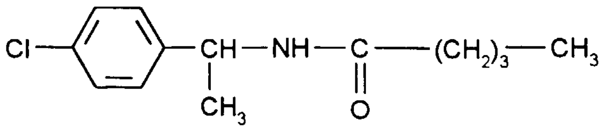

(I-4)

Ein Gemisch aus 2,4 g (0,01 Mol) (S)-N-[1-(4-Chlor-phenyl)-ethyl]-valeriansäureamid ($[\alpha]_D^{20}$ = -105,1° c = 1 in Methanol), 0,27 g (0,004 Mol) Kaliumhydroxid und 15 ml Dimethylsulfoxid wird 32 Stunden lang auf 100°C erhitzt. Danach wird das Reaktionsgemisch unter vermindertem Druck eingeengt und der verbleibende Rückstand mit 50 ml Dichlormethan versetzt. Man wäscht das entstehende Gemisch dreimal mit je 20 ml Wasser, trocknet über Natriumsulfat und engt unter vermindertem Druck ein.

Man erhält auf diese Weise 1,96 g an N-[1-(4-Chlor-phenyl)-ethyl]-valeriansäureamid ($[\alpha]_D^{20}$ = ±0° c = 1,141 in Methanol). Die Ausbeute an racemischem Produkt errechnet sich danach zu 81,7 % der Theorie.

**Beispiel 11**

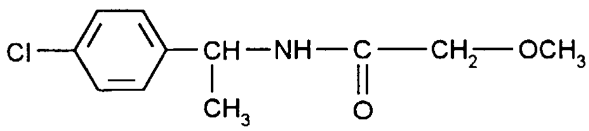

(I-5)

Ein Gemisch aus 2,3 g (0,01 Mol) (S)-N-[1-(4-Chlor-phenyl)-ethyl]-2-methoxyacetamid ($[\alpha]_D^{20}$ = -78,9° c = 1 in Methanol), 0,33 g (0,005 Mol) Kaliumhydroxid und 15 ml Dimethylsulfoxid wird 32 Stunden lang auf 100°C erhitzt. Danach wird in der im Beispiel 10 angegebenen Weise aufgearbeitet. Man erhält auf diese Weise 1,96 g an N-[1-(4-Chlor-phenyl)-ethyl]-2-methoxy-acetamid ($[\alpha]_D^{20}$ = ±0° c = 1,14 in Methanol). Die Ausbeute an racemischem Produkt errechnet sich danach zu 85,2 % der Theorie.

**Beispiel 12**

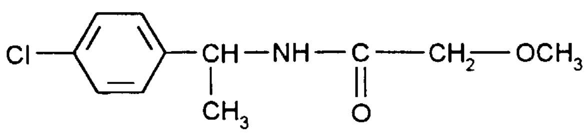

(I-5)

Ein Gemisch aus 2,3 g (0,01 Mol) (S)-N-[1-(4-Chlor-phenyl)-ethyl]-2-methoxyacetamid ($[\alpha]_D^{20}$ = -78,9° c = 1 in Methanol), 0,56 g (0,005 Mol) Kalium-tert.-butanolat und 15 ml Dimethylsulfoxid wird 32 Stunden lang auf 100°C erhitzt. Danach wird in der im Beispiel 9 angegebenen Weise aufgearbeitet. Man erhält auf diese Weise 1,95 g an N-[1-(4-Chlor-phenyl)-ethyl]-2-methoxy-acetamid ($[\alpha]_D^{20}$ = -2,9° c = 1,01 in Methanol). Die Ausbeute an racemischem Produkt errechnet sich danach zu 85,2 % der Theorie.

**Patentansprüche**

1. Verfahren zur Herstellung von racemischen Amino-Derivaten der Formel

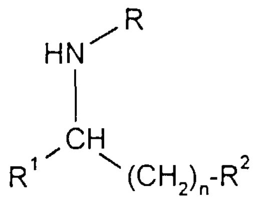

(I),

in welcher

n für die Zahlen 0, 1 oder 2 steht,

$R^1$ für gegebenenfalls substituiertes Alkyl, gegebenenfalls substituiertes Cycloalkyl, gegebenenfalls substituiertes Cycloalkenyl, gegebenenfalls substituiertes Aryl oder gegebenenfalls substituiertes Heterocyclyl steht,

$R^2$ für gegebenenfalls substituiertes Alkyl, gegebenenfalls substituiertes Cycloalkyl, gegebenenfalls substituiertes Cycloalkenyl, gegebenenfalls substituiertes Aryl oder gegebenenfalls substituiertes Heterocyclyl steht, wobei jedoch $R^1$ und $R^2$-$(CH_2)_n$- nicht identisch sind, und

R für Wasserstoff oder -CO-$R^3$ steht, worin

$R^3$ für Wasserstoff, Amino, für gegebenenfalls substituiertes Alkyl oder für gegebenenfalls substituiertes Alkoxy steht, wobei die Kohlenstoffkette bei denjenigen Resten, die mehr als ein Kohlenstoffatom enthalten, durch Heteroatome oder - gruppen, unterbrochen sein kann, oder

$R^3$ für gegebenenfalls substituiertes Cycloalkyl, gegebenenfalls substituiertes Cycloalkenyl, gegebenenfalls substituiertes Aryl oder gegebenenfalls substituiertes Heterocyclyl steht,

dadurch gekennzeichnet, daß man optisch aktive Amide der Formel

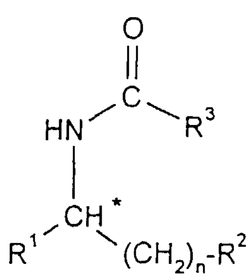

(II),

in welcher

$R^1$, $R^2$, $R^3$ und n die oben angegebenen Bedeutungen haben,

mit starken Basen aus der Gruppe der Erdalkalimetall- oder Alkalimetallhydride, -hydoxide, -amide oder -alkoholate, gegebenenfalls in Gegenwart eines organischen Verdünnungsmittels umsetzt und gegebenenfalls die dabei entstehenden racemischen Amide der Formel

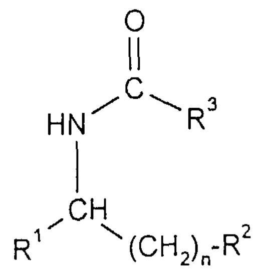

(Ia),

in welcher

$R^1$, $R^2$, $R^3$ und n die oben angegebenen Bedeutungen haben,

entweder

a) mit Wasser in Gegenwart einer Base oder einer Säure und gegebenenfalls in Gegenwart eines organischen Verdünnungsmittels umsetzt,

oder

b) sofern es sich bei den Verbindungen der Formel (Ia) um Amide handelt, in denen $R^3$ für Wasserstoff steht, mit Alkalimetallhydroxid oder Erdalkalimetallhydroxid umsetzt.

**2.** Verfahren gemäß Anspruch 1, dadurch gekennzeichnet, daß man optisch aktive Amide der Formel (II) einsetzt, in denen

n für die Zahlen 0, 1 oder 2 steht,

$R^1$ für Methyl, Ethyl, n-Propyl oder Isopropyl steht,

$R^2$ für Cyclohexyl, Norbornyl oder Cyclohexenyl steht, wobei diese Reste einfach bis vierfach, gleichartig oder verschieden substituiert sein können durch Fluor, Chlor, Methyl und/oder Ethyl, oder
für Furyl, Pyridyl oder Thienyl steht, wobei diese Reste einfach bis dreifach, gleichartig oder verschieden substituiert sein können durch Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, Trifluormethyl und/oder Trifluorethyl, oder
für Phenyl oder Naphthyl steht, wobei jeder dieser Reste einfach bis fünffach, gleichartig oder verschieden substituiert sein kann durch Fluor, Chlor, Brom, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Methoxycarbonyl, Ethoxycarbonyl, Phenyl, Phenoxy, Phenylthio, Benzyloxy, Benzylthio oder Phenylethyloxy, oder durch jeweils gegebenenfalls einfach oder mehrfach, gleich oder verschieden durch Fluor, Chlor, Methyl, Trifluormethyl, Ethyl, n-oder i-Propyl substituiertes, jeweils zweifach verknüpftes Trimethylen (Propan-1,3-diyl), Methylendioxy oder Ethylendioxy, und

$R^3$ für Wasserstoff, Amino, Methyl, Ethyl, n- oder i-Propyl, n-, i-, s-Butyl, Pentyl, Hexyl, Heptyl, Octyl, Methoxy, Ethoxy, Hydroxymethyl, 1-Hydroxy-1-ethyl, Methoxymethyl, Methoxyethyl, Methoxypropyl, Ethoxymethyl, Ethoxyethyl, 2-Carboxy-1-hydroxy-1-ethyl, 2-Carboxy-1-ethyl, 3-Carboxy-1-propyl und Benzyl steht.

**3.** Verfahren gemäß Anspruch 1, dadurch gekennzeichnet, daß man als optisch aktives Amid der Formel (II) (S)-N-[1-(4-Chlor-phenyl)-ethyl]-acetamid der Formel

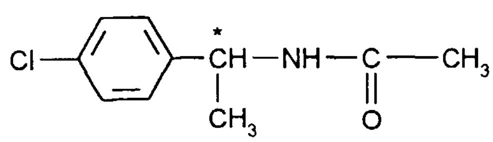

einsetzt.

**4.** Verfahren gemäß Anspruch 1, dadurch gekennzeichnet, daß man als optisch aktives Amid der Formel (II) (S)-N-[1-(4-Chlor-phenyl)-ethyl]-formamid der Formel

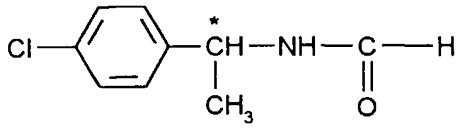

einsetzt.

**5.** Verfahren gemäß Anspruch 1, dadurch gekennzeichnet, daß man als optisch aktives Amid der Formel (II) (S)-N-[1-(4-Chlor-phenyl)-ethyl]-valeriansäureamid der Formel

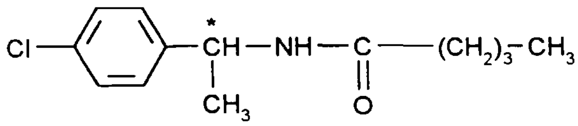

einsetzt.

**6.** Verfahren gemäß Anspruch 1, dadurch gekennzeichnet, daß man als optisch aktives Amid der Formel (II) (S)-N-[1-(4-Chlor-phenyl)-ethyl]-2-methoxyacetamid der Formel

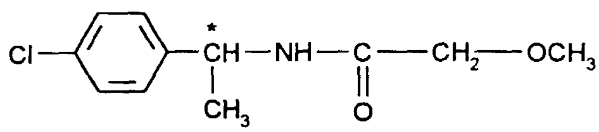

einsetzt.

**7.** Verfahren gemäß Anspruch 1, dadurch gekennzeichnet, daß man als optisch aktives Amid der Formel (II) (S)-N-[1-(4-Chlor-phenyl)-ethyl]-benzylamid der Formel

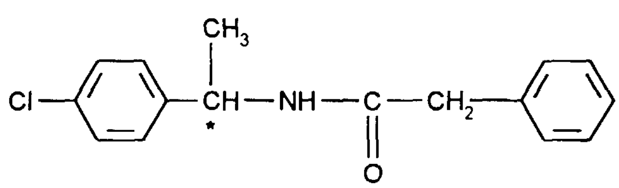

einsetzt.

**8.** Verfahren gemäß Anspruch 1, dadurch gekennzeichnet, daß man bei der Durchführung der ersten Stufe bei Temperaturen zwischen 0°C und 280°C arbeitet und bei der Durchführung der zweiten Stufe bei Temperaturen zwischen 0°C und 200°C arbeitet.

**9.** Verfahren gemäß Anspruch 1, dadurch gekennzeichnet, daß man bei der Durchführung der ersten Stufe auf 1 Mol an optisch aktivem Amid der Formel (II) 0,01 bis 2 Mol an starker Base einsetzt.

**10.** Verfahren gemäß Anspruch 1, dadurch gekennzeichnet daß man bei der Durchführung der zweiten Stufe nach

Variante (b) Kaliumhydroxid als Base einsetzt.

## Claims

1. Process for preparing racemic amino derivatives of the formula

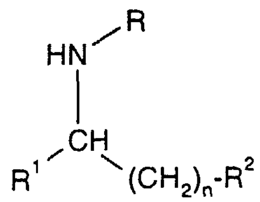

(I),

where

n represents the number 0, 1 or 2,

$R^1$ represents unsubstituted or substituted alkyl, unsubstituted or substituted cycloalkyl, unsubstituted or substituted cycloalkenyl, unsubstituted or substituted aryl or unsubstituted or substituted heterocyclyl,

$R^2$ represents unsubstituted or substituted alkyl, unsubstituted or substituted cycloalkyl, unsubstituted or substituted cycloalkenyl, unsubstituted or substituted aryl or unsubstituted or substituted heterocyclyl, but with $R^1$ and $R^2$-$(CH_2)_n$- not being identical, and

R represents hydrogen or -CO-$R^3$, where

$R^3$ represents hydrogen, amino, unsubstituted or substituted alkyl or unsubstituted or substituted alkoxy, with the carbon chain being able, in the case of those radicals containing more than one carbon atom, to be interrupted by heteroatoms or hetero groups, or

$R^3$ represents unsubstituted or substituted cycloalkyl, unsubstituted or substituted cycloalkenyl, unsubstituted or substituted aryl or unsubstituted or substituted heterocyclyl,

characterized in that optically active amides of the formula

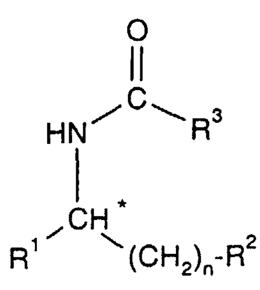

(II),

where

$R^1$,$R^2$,$R^3$ and n are as defined above,

are reacted with strong bases from the group consisting of the alkaline earth metal hydrides, hydroxides, amides or alcoholates or alkali metal hydrides, hydroxides, amides or alcoholates, optionally in the presence of an organic diluent and, if desired, the resulting racemic amides of the formula

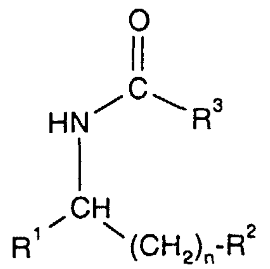

(Ia),

where

$R^1$,$R^2$,$R^3$ and n are as defined above,

are reacted either

a) with water in the presence of a base or an acid and optionally in the presence of an organic diluent,

or

b) if the compounds of the formula (Ia) are amides in which $R^3$ is hydrogen, with alkali metal hydroxide or alkaline earth metal hydroxide.

**2.** Process according to Claim 1, characterized in that the optically active amides of the formula (II) which are used are ones in which

n represents the number 0, 1 or 2,

$R^1$ represents methyl, ethyl, n-propyl or isopropyl,

$R^2$ represents cyclohexyl, norbornyl or cyclohexenyl, with these radicals being able to be monosubstituted to tetrasubstituted, identically or differently, by fluorine, chlorine, methyl and/or ethyl, or represents furyl, pyridyl or thienyl, with these radicals being able to be monosubstituted to trisubstituted, identically or differently, by fluorine, chlorine, bromine, methyl, ethyl, n-propyl, i-propyl, n-butyl, i-butyl, s-butyl, t-butyl, methoxy, ethoxy, n-propoxy, i-propoxy, trifluoromethyl and/or trifluoroethyl, or represents phenyl or naphthyl, with each of these radicals being able to be monosubstituted to pentasubstituted, identically or differently, by fluorine, chlorine, bromine, nitro, arnino, hydroxy, formyl, carboxy, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, methoxycarbonyl, ethoxycarbonyl, phenyl, phenoxy, phenylthio, benzyloxy, benzylthio or phenylethyloxy, or by in each case doubly linked trimethylene (propane-1,3-diyl), methylenedioxy or ethylenedioxy which may each be unsubstituted, monosubstituted or polysubstituted, identically or differently, by fluorine, chlorine, methyl, trifluoromethyl, ethyl, n- or i-propyl, and

$R^3$ represents hydrogen, amino, methyl, ethyl, n- or i-propyl, n-, i-, s-butyl, pentyl, hexyl, heptyl, octyl, methoxy, ethoxy, hydroxymethyl, 1-hydroxy-1-ethyl, methoxymethyl, methoxyethyl, methoxypropyl, ethoxymethyl, ethoxyethyl, 2-carboxy-1-hydroxy-1-ethyl, 2-carboxy1-ethyl, 3-carboxy-1-propyl or benzyl.

**3.** Process according to Claim 1, characterized in that the optically active amide of the formula (II) which is used is (S)-N-[1-(4-chloro-phenyl)-ethyl]-acetamide of the formula

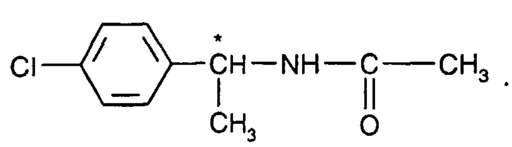

**4.** Process according to Claim 1, characterized in that the optically active amide of the formula (II) which is used is (S)-N-[1-(4-chloro-phenyl)-ethyl]-formamide of the formula

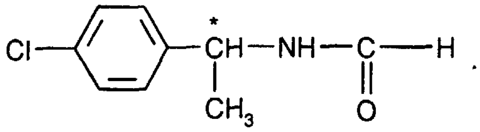

**5.** Process according to Claim 1, characterized in that the optically active amide of the formula (II) which is used is (S)-N-[1-(4-chloro-phenyl)-ethyl]-valeramide of the formula

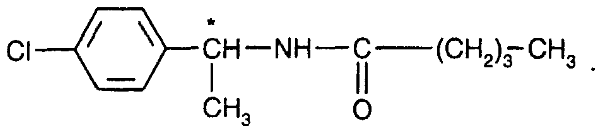

**6.** Process according to Claim 1, characterized in that the optically active amide of the formula (II) which is used is (S)-N-[1-(4-chloro-phenyl)-ethyl]-2-methoxy-acetamide of the formula

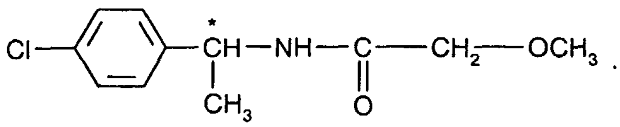

**7.** Process according to Claim 1, characterized in that the optically active amide of the formula (II) which is used is (S)-N-[1-(4-chloro-phenyl)-ethyl]-benzylamide of the formula

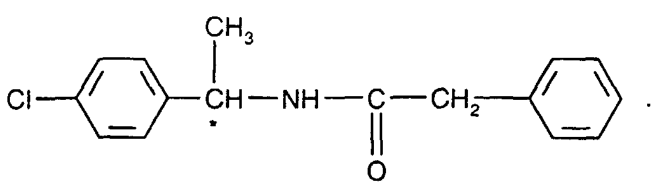

**8.** Process according to Claim 1, characterized in that the first stage is carried out at temperatures of between 0°C and 280°C and the second Stage is carried out at temperatures of between 0°C and 200°C.

**9.** Process according to Claim 1, characterized in that the first stage is carried out using from 0.01 to 2 mol of strong base per 1 mol of optically active amide of the formula (II).

**10.** Process according to Claim 1, characterized in that the second stage according to variant (b) is carried out using potassium hydroxide as base.

**Revendications**

**1.** Procédé pour la préparation de dérivés amino racémiques répondant à la formule

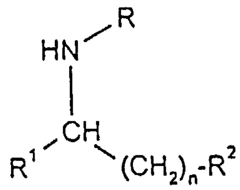

(I),

dans laquelle

n représente les nombres 0, 1 ou 2,

$R^1$ représente un groupe alkyle le cas échéant substitué, un groupe cycloalkyle le cas échéant substitué, un groupe cycloalcényle le cas échéant substitué, un groupe aryle le cas échéant substitué ou un groupement hétérocyclyle le cas échéant substitué,

$R^2$ représente un groupe alkyle le cas échéant substitué, un groupe cycloalkyle le cas échéant substitué, un groupe cycloalcényle le cas échéant substitué, un groupe aryle le cas échéant substitué ou un groupement hétérocyclyle le cas échéant substitué; toutefois $R^1$ et $R^2$-$(CH_2)_n$ ne sont pas identiques, et

R représente un atome d'hydrogène ou un groupe -CO-$R^3$ dans lequel

$R^3$ représente un atome d'hydrogène, un groupe amino, un groupe alkyle le cas échéant substitué ou un groupe alcoxy le cas échéant substitué, la chaîne carbonée pour des radicaux qui contiennent plus d'un atome de carbone pouvant être interrompue par des hétéroatomes ou par des hétérogroupes, ou

$R^3$ représente un groupe cycloalkyle le cas échéant substitué, un groupe cycloalcényle le cas échéant substitué, un groupe aryle le cas échéant substitué, un groupement hétérocyclyle le cas échéant substitué,

caractérisé en ce qu'on fait réagir des amides optiquement actifs répondant à la formule

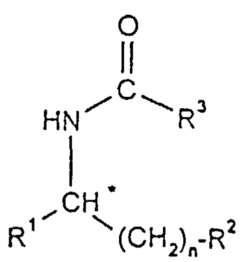

(II),

dans laquelle

$R^1$, $R^2$, $R^3$ et n ont les significations indiquées ci-dessus,

avec des bases fortes choisies parmi le groupe comprenant des hydrures, des hydroxydes, des amidures ou des alcoolates de métaux alcalino-terreux ou de métaux alcalins,

le cas échéant en présence d'un diluant organique et, le cas échéant, on fait réagir les amides racémiques obtenus en l'occurrence, répondant à la formule

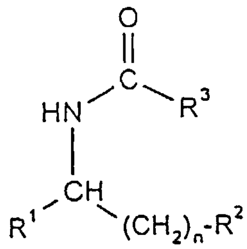

(Ia),

dans laquelle

$R^1$, $R^2$, $R^3$ et n ont les significations indiquées ci-dessus,

soit

a) avec de l'eau en présence d'une base ou d'un acide et le cas échéant en présence d'un diluant organique,

soit

b) pour autant qu'il s'agisse, en ce qui concerne les composés répondant à la formule (Ia), d'amides dans lesquels $R^3$ représente un atome d'hydrogène, avec un hydroxyde de métal alcalin ou avec un hydroxyde de métal alcalino-terreux.

**2.** Procédé selon la revendication 1, caractérisé en ce qu'on met en oeuvre des amides optiquement actifs répondant à la formule (II), dans lesquels

n représente les nombres 0, 1 ou 2,

$R^1$ représente un groupe méthyle, un groupe éthyle, un groupe n-propyle ou un groupe isopropyle,

$R^2$ représente un groupe cyclohexyle, un groupe norbornyle ou un groupe cyclohexényle, ces radicaux pouvant porter de un à quatre substituants identiques ou différents fluoro, chloro, méthyle et/ou éthyle, ou représente un groupe furyle, un groupe pyridyle ou un groupe thiényle, ces radicaux pouvant porter de un à trois substituants identiques ou différents fluoro, chloro, bromo, méthyle, éthyle, n-propyle, i-propyle, n-butyle, i-butyle, s-butyle, t-butyle, méthoxy, éthoxy, n-propoxy, i-propoxy, trifluorométhyle et/ou trifluoréthyle, ou représente un groupe phényle ou un groupe naphtyle, chacun de ces radicaux pouvant porter de un à cinq substituants identiques ou différents fluoro, chloro, bromo, nitro, amino, hydroxyle, formyle, carboxyle, carbamoyle, thiocarbamoyle, méthyle, éthyle, n- ou i-propyle, n-, i-, s- ou t-butyle, méthoxy, éthoxy, n- ou i-propoxy, méthylthio, éthylthio, n- ou i-propylthio, méthylsulfinyle, éthylsulfinyle, méthylsulfonyle ou éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle ou trifluorométhylsulfonyle, méthylamino, éthylamino, n- ou i-propylamino, diméthylamino, diéthylamino, méthoxycarbonyle, éthoxycarbonyle, phényle, phénoxy, phénylthio, benzyloxy, benzylthio ou phényléthyloxy, ou encore de un à cinq substituants triméthylène (propane-1,3-diyle), méthylènedioxy ou éthylènedioxy, chacun de ces substituants étant doublement lié et portant le cas échéant respectivement un ou plusieurs substituants identiques ou différents fluoro, chloro, méthyle, trifluorométhyle, éthyle, n- ou i-propyle, et

$R^3$ représente un atome d'hydrogène, un groupe amino, un groupe méthyle, un groupe éthyle, un groupe n- ou i-propyle, un groupe n-, i-, s-butyle, un groupe pentyle, un groupe hexyle, un groupe heptyle, un groupe octyle, un groupe méthoxy, un groupe éthoxy, un groupe hydroxyméthyle, un groupe 1-hydroxy-1-éthyle, un groupe méthoxyméthyle, un groupe méthoxyéthyle, un groupe méthoxypropyle, un groupe éthoxyméthyle, un groupe éthoxyéthyle, un groupe 2-carboxy-1-hydroxy-1-éthyle, un groupe 2-carboxy-1-éthyle, un groupe 3-carboxy-1-propyle et un groupe benzyle.

**3.** Procédé selon la revendication 1, caractérisé en ce qu'on met en oeuvre, à titre d'amide optiquement actif répondant à la formule (II), le (S)-N-[1-(4-chlorophényl)-éthyl]-acétamide répondant à la formule

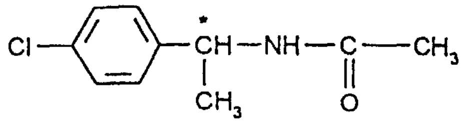

4. Procédé selon la revendication 1, caractérisé en ce qu'on met en oeuvre, à titre d'amide optiquement actif répondant à la formule (II), le (S)-N-[1-(4-chlorophényl)-éthyl]-formamide répondant à la formule

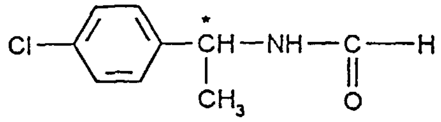

5. Procédé selon la revendication 1, caractérisé en ce qu'on met en oeuvre, à titre d'amide optiquement actif répondant à la formule (II), l'amide de l'acide (S)-N-[1-(4-chlorophényl)-éthyl]-valérianique répondant à la formule

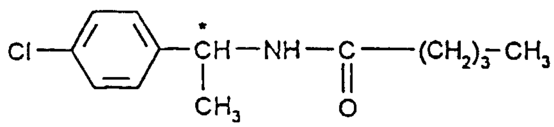

6. Procédé selon la revendication 1, caractérisé en ce qu'on met en oeuvre, à titre d'amide optiquement actif répondant à la formule (II), le (S)-N-[1-(4-chlorophényl)-éthyl]-2-méthoxyacétamide répondant à la formule

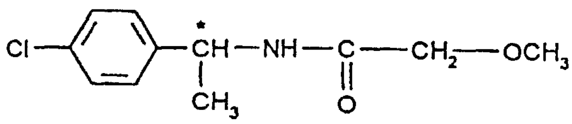

7. Procédé selon la revendication 1, caractérisé en ce qu'on met en oeuvre, à titre d'amide optiquement actif répondant à la formule (II), le (S)-N-[1-(4-chlorophényl)-éthyl]-benzylamide répondant à la formule

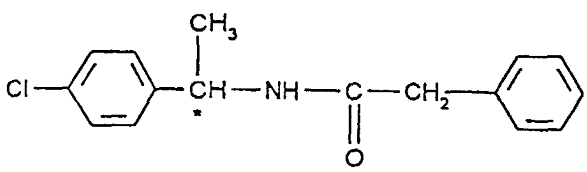

8. Procédé selon la revendication 1, caractérisé en ce qu'on travaille, lorsqu'on effectue la première étape, à des températures entre 0°C et 280°C, et lorsqu'on effectue la seconde étape, à des températures entre 0°C et 200°C.

9. Procédé selon la revendication 1, caractérisé en ce qu'on met en oeuvre, lorsqu'on effectue la première étape, pour 1 mole d'amide optiquement actif répondant à la formule (II), de 0,01 à 2 moles d'une base forte.

10. Procédé selon la revendication 1, caractérisé en ce qu'on met en oeuvre, lorsqu'on effectue la seconde étape conformément à la variante (b), de l'hydroxyde de potassium à titre de base.